# EUROPÄISCHE PATENTSCHRIFT

(11) **EP 1 309 310 B1**
(45) Veröffentlichungstag und Bekanntmachung des Hinweises auf die Patenterteilung: **11.05.2005**
(21) Anmeldenummer: 01962631.6
(22) Anmeldetag: 14.08.2001
(51) Int. Cl.: A61K 7/48, A61K 7/46

(54) **ALKOHOLISCHES BASISGEMISCH FÜR DUFTZUBEREITUNGEN**
ALCOHOL BASE MIXTURE FOR PERFUMED PREPARATIONS
MELANGE DE BASE ALCOOLISE POUR PREPARATIONS PARFUMEES

(30) Priorität: 15.08.2000 DE 10042575
(43) Veröffentlichungstag der Anmeldung: 14.05.2003
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC); OLIVIER, Doucet, F-06230 Villefranche s/Mer (FR)
(74) Vertreter: Walter, Wolf-Jürgen
(86) Internationale Anmeldenummer: PCT/DE2001/003070
(87) Internationale Veröffentlichungsnummer: WO 2002/013769

(56) Entgegenhaltungen:
- EP-A- 0 875 236
- EP-A- 1 106 174
- DE-A- 19 941 819

## Beschreibung

Die Erfindung betrifft ein alkoholisches Basisgemisch mit Extrakten von Früchten und Milchprodukten.

Es sind bereits eine Reihe von Vorschlägen gemacht worden, Früchte oder Fruchtextrakte einschließlich maritimer Pflanzen und Algen in kosmetischen Produkten zu verarbeiten (DE-C-3515231 Hafnia-Extrakt; DE-A-19824727 Catechin und grüner Tee).

Es ist weiterhin bekannt, Milch oder aufbereitete Milchprodukte in kosmetische Produkte einzubringen. So ist aus der EP-A-839519 eine protein- und vitaminhaltige Kosmetikzusammensetzung bekannt, die 5-90 Gew-% Säugermilch, 1-6 Gew-% Protein und Hefeglucan enthält.

Aufgabe der vorliegenden Erfindung ist es, ein Basisgemisch für Duftzubereitungen zu entwickeln, das spezielle Hautwirksamkeiten aufweist.

Erfindungsgemäß bereitgestellt wird ein alkoholisches Basisgemisch für Duftzubereitungen, das dadurch gekennzeichnet ist, daß es aus dem Extrakt einer alkoholischen Extraktion im Temperaturbereich 10 bis 30 °C von Ananas-Früchten und dem Extrakt einer alkoholischen Extraktion bei 10 bis 30 °C von Joghurt besteht, wobei das Verhältnis Ananas-Extrakt zu Joghurtextrakt im Bereich von 20:80 bis 80:20 liegt, bezogen auf das Gewicht.

Ein bevorzugtes Verhältnis der beiden Extrakte liegt im Bereich von 40:60 bis 60:40.

Der Extrakt der Ananas-Frucht wird durch Extraktion mit Alkohol bei einer Temperatur im Bereich von 10 bis 30 °C, vorzugsweise 15 bis 25 °C erhalten. Dabei geht man von zerkleinerten frischen Früchten aus, die unter Bewegung über einen Zeitraum von 5 bis 36 Stunden mit Alkohol, z.B. Ethylakohol in Kontakt gebracht werden. Der Extrakt wird anschließend filtriert, um die Feststoffanteile abzutrennen.

Durch die intensive Extraktion werden eine Reihe alkohollöslicher Wirkstoffe gelöst, nicht jedoch die in der Ananas vorhandenen Enzyme. Auch Teile der enthaltenen Folsäure gehen in den Extrakt über.

Als zweite Komponente wird reiner Naturjoghurt aus Milch von Kühen, Schafen, Ziegen oder anderen Tieren oder Gemischen von verschiedenen Milcharten, wird ebenfalls mit Alkohol, z.B. Ethylalkohol bei 10 bis 30 °C, vorzugsweise 15-25 °C, extrahiert über einen Zeitraum von 5 bis 36 Stunden unter intensiver Bewegung. Anschließend wird die alkoholische dünnflüssige Phase z.B. durch mehrfache Filtration rückstandsfrei abgetrennt. Dieser filtrierte Extrakt wird dann einer Sterilisation unterworfen. Die im Extrakt enthaltenen Wirkstoffe, u.a. auch Vitamine, bleiben für die Weiterverwendung erhalten. Der alkoholische Joghurtextrakt wird mit dem alkoholischen Ananas-Extrakt, die beide klare Lösungen darstellen, vermischt.

Die alkolischen Extrakte sind die Basis für Duftzubereitungen wie Parfüms, Sprays oder Duftöle und gegebenenfalls Duftkerzen. Besonders bevorzugt sind Parfüme. Erfindungsgemäß enthält ein solches Parfüm über die sonst in einem Parfüm enthaltenen Duftkomponenten hinaus das in einer Konzentration von 0,5 bis 10 Gew-% zugesetzte erfindungsgemäße Gemisch mit ungewöhnlichen Wirkstoffe der Ananas und von Joghurt.

Diese Wirkstoffe entfalten nach dem Auftragen auf die Haut ihre Wirkung. Wesentliche spezielle Wirkung ist - in Abhängigkeit von der aufgetragenen Menge bzw. von der Größe der dafür vorgesehenen Körperfläche - ein "Happyness"-Gefühl. Darunter ist nicht ein reines Glücksgefühl zu verstehen, sondern eine ausgewogene Zufriedenheit, eine Entspannung gepaart mit angenehmen Empfindungen. Ohne an eine Theorie gebunden zu sein, scheint sich die Aufnahme des Produktes über die Haut auf den Serotinspiegel und die Noradrenalinbildung auszuwirken.

Die bei dem Ananas-Extrakt mögliche helle Gelbfärbung kann durch Zusätze in dem fertigen kosmetischen Produkt, wie einem Parfüm, durch gelbe Farbstoffe noch verstärkt werden. Es ist bekannt, daß durch die Farbe Gelb auch eine stimulierung von Glücksgefühlen hervorgerufen werden kann, die zur Gesamtwirkung eines Parfüms noch beitragen kann und insbesondere das Wohlbefinden der Trägerin/des Trägers verstärken kann.

Die Duftzubereitung mit dem erfindungsgemäßen Gemisch kann Trägerstoffe wie Alkohole, Öle, Ester, Treibmittel enthalten.

Die Erfindung soll nachstehend durch Beispiele näher erläutert werden. Alle Angaben erfolgen in Gewichtsprozent, sofern nichts anderes angegeben ist.

### Beispiel 1 Parfüm

| | |
|---|---|
| Parfümöl | 13 |
| Ethanol | ad 100 |
| Basisgemisch mit Ananasextrakt: | |
| Joghurtextrakt 55:45 | 2 |
| Farbstoff gelb | 0,1 |

Die genannten Komponenten werden miteinander verrührt. Anschließend läßt man das Gemisch reifen und filtriert dann ab.

### Beispiel 2

Die Herstellung des Basisgemisches erfolgt durch Vermischen etwa gleicher Gewichtsteile Alkohol und frischer, geschälter und zerkleinerter Ananasstücken. Das Mischen wird über einen Zeitraum von 18 Stunden bei 20 °C fortgeführt. Anschließend wird das Gemisch filtriert und das Filtrat als eine Ausgangskomponente des Basisgemisches eingesetzt.

Weiterhin werden 60 Masseteile Ethylalkohol und 40 Masseteile eines frischen Naturjoghurt aus Kuhmilch bei 20 °C für einen Zeitraum von 20 Stunden unter intensivem Rühren miteinander vermischt und das Gemisch anschließend abfiltriert. Das Filtrat wird noch wenigstens zweimal und zum Schluß durch ein Millipore-Filter filtriert.

Die beiden Extrakte, die nunmehr klare Lösungen darstellen, werden im gewünschten Verhältnis miteinander vermischt und stellen das Basigemisch dar.

## Patentansprüche

1. Alkoholisches Basisgemisch für Duftzubereitungen, **dadurch gekennzeichnet, daß** es aus dem Extrakt einer alkoholischen Extraktion im Temperaturbereich 10 bis 30 °C von Ananas-Früchten und dem Extrakt einer alkoholischen Extraktion im Temperaturbereich 10 bis 30 °C von Joghurt besteht, wobei das Verhältnis Ananas-Extrakt zu Joghurtextrakt im Bereich von 20:80 bis 80:20 liegt.

2. Gemisch nach Anspruch 1, **dadurch gekennzeichnet, daß** der Alkoholanteil des Gemisches aus Ethylakohol besteht.

3. Gemisch nach Anspruch 1, **dadurch gekennzeichnet, daß** das Verhältnis der beiden Extrakte im Bereich von 40:60 bis 60:40 liegt.

4. Gemisch nach Anspruch 1, **dadurch gekennzeichnet, daß** es in einer Duftzubereitung in einem Anteil von 0,5 bis 10 Gew-% vorliegt, bezogen auf die Gesamtmasse der Zubereitung.

5. Gemisch nach Anspruch 1, **dadurch gekennzeichnet, daß** die Duftzubereitung ein Parfüm, ein Spray oder ein Duftöl ist, insbesondere ein Parfüm ist.

6. Gemisch nach Anspruch 1, **dadurch gekennzeichnet, daß** die Duftzubereitung Trägerstoffe wie Alkohole, Öle, Ester, Treibmittel enthält.

7. Gemisch nach Anspruch 1, **dadurch gekennzeichnet, daß** die Duftzubereitung eine helle Gelbfärbung aufweist.

## Claims

1. An alcoholic base mixture for perfumed preparations which comprises the extract from pineapple fruit obtained by alcoholic extraction in the temperature range from 10 to 30 °C and the extract from yogurt obtained by alcoholic extraction in the temperature range from 10 to 30 °C, the ratio of pineapple extract to yogurt extract ranging from 20:80 to 80:20.

2. A mixture according to Claim 1 wherein the alcohol content of the mixture consists of ethyl alcohol.

3. A mixture according to Claim 1 wherein the ratio of the two extracts is in the range from 40:60 to 60:40.

4. A mixture according to Claim 1 wherein the said mixture is contained in a perfumed preparation in an amount ranging from 0.5 to 10 % by weight relative to the total weight of the preparation.

5. A mixture according to Claim 1 wherein the perfumed preparation is a perfume, a spray or a perfumed oil, particularly a perfume.

6. A mixture according to Claim 1 wherein the perfumed preparation contains carrier substances such as alcohols, oils, esters, propellants.

7. A mixture according to Claim 1 wherein the perfumed preparation has a bright yellow coloration.

## Revendications

1. Mélange de base pour préparations parfumées, **caractérisé en ce qu'**il se compose de l'extrait d'une extraction alcoolique de fruits d'ananas dans le domaine de température de 10 à 30°C et de l'extrait d'une extraction alcoolique de yaourt dans le domaine de température de 10 à 30°C, la proportion d'extrait d'ananas à l'extrait de yaourt se trouvant dans le domaine de 20:80 à 80:20.

2. Mélange selon la revendication 1, **caractérisé en ce que** la portion d'alcool du mélange se compose d'alcool éthylique.

3. Mélange selon la revendication 1, **caractérisé en ce que** la proportion des deux extraits se trouve dans le domaine de 40:60 à 60:40.

4. Mélange selon la revendication 1, **caractérisé en ce qu'**il se trouve dans une préparation parfumée dans une proportion de 0,5 à 10 % en poids, par rapport au poids total de la préparation.

5. Mélange selon la revendication 1, **caractérisé en ce que** la préparation parfumée est un parfum, un aérosol ou une huile parfumée, en particulier un parfum.

6. Mélange selon la revendication 1, **caractérisé en ce que** la préparation parfumée contient des substances porteuses telles que des alcools, des huiles, des esters ou des substances propulsives.

7. Mélange selon la revendication 1, **caractérisé en ce que** la préparation parfumée présente une coloration jaune pale.
